# EUROPEAN PATENT APPLICATION

(11) **EP 1 570 845 A1**
(43) Date of publication of application: **07.09.2005**
(21) Application number: 03780683.3
(22) Date of filing: 05.12.2003
(51) Int. Cl.: A61K 31/216, A61K 31/737, A61K 31/78, A61K 35/80, A61P 19/08, A61P 19/10, A61P 43/00

(54) **REMEDY**

(30) Priority: 05.12.2002 JP 2002354356; 26.12.2002 JP 2002378699
(71) Applicant: TAKARA BIO INC., Shiga 520-2193 (JP)
(72) Inventor: ONOGI, Hiromu, Kusatsu-shi, Shiga 525-0025 (JP); SUGIYAMA, Katsumi, Otsu-shi, Shiga 520-2134 (JP); SAGAWA, Hiroaki, Kusatsu-shi, Shiga 525-0025 (JP); KATO, Ikunoshin, Koka-shi, Shiga 529-1851 (JP)
(74) Representative: Vossius & Partner
(86) International application number: PCT/JP2003/015576
(87) International publication number: WO 2004/050078

(57) **Abstract**

The present invention relates to a therapeutic agent or prophylactic agent for a disease requiring enhancement of bone morphogenetic protein production or promotion of osteogenesis; an agent for enhancement of bone morphogenetic protein production or an agent for promotion of osteogenesis, a food, beverage or feed for enhancement of bone morphogenetic protein production or promotion of osteogenesis, **characterized in that** each comprises as an effective ingredient at least one compound selected from the group consisting of (a) an acidic saccharide, (b) a polyacrylic acid, (c) chlorogenic acid and (d) an oxidation processed product of chlorogenic acid, or an extract derived from algae.

## Description

### TECHNICAL FIELD

The present invention relates to a medicament, food, beverage or feed, which is useful for the treatment or prevention of a disease requiring enhancement of bone morphogenetic protein production or promotion of osteogenesis, for instance, osteoporosis, bone fracture or the like.

### BACKGROUND ART

In osseous tissues, osteogenesis and bone resorption are always repeated with maintaining a constant balance therebetween, thereby regulating bone strength and calcium concentration in blood. In the osteogenesis, osteoblasts play a key role, and in the bone resorption, osteoclasts play a key role. And it is considered that the osteoporosis is caused when the balance between the osteogenesis and the bone resorption is lost for some sort of reasons. The osteoporosis is roughly classified into postmenopausal osteoporosis caused by the lowering of estrogen secretion, and senile osteoporosis caused by aging. Besides these, secondary osteoporosis caused by an incretion or metabolism disease such as diabetes or hyperthyrea, administration of a drug such as a steroid, a disease in the digestive organ or the liver, vitamin C deficiency, immobility, oophorectomy, articular rheumatism, or the like has been known.

At present, as a therapeutic agent for osteoporosis, a drug for suppressing quantitative loss of the bone by mainly inhibiting bone resorption, such as an estrogen agent, calcitonin, or a bisphosphonate has been used. However, there are some drawbacks in the treatment with the estrogen agent, there is a strong adverse action such as an increase risk in breast cancer, uterine cancer, or a cardiac disease, in the treatment with calcitonin, the resistance to the drug is easily generated, thereby making it impossible to orally administer the drug, and in the treatment with the bisphosphonate, the absorption ratio is worsened, so that residuality is so high that excess inactivation of bone metabolism is undesirably caused. Also, a vitamin D₃ preparation has been used for the purpose of activating bone metabolism. However, its adverse action such as hypercalcemia is large while its therapeutic effect is smaller than other drugs. These conventional therapeutic agents for osteoporosis cannot recover the already progressed bone defects to the original state, so that the agents can be hardly said to be satisfactory as true therapeutic agents for osteoporosis.

Cells closely relating to the osteogenesis are osteoblasts. The osteoblasts are originated from mesenchymal stem cells which are common with cartilage cells, muscle cells, fat cells, tendon cells or the like. In the process of differentiation, the osteoblasts become mature osteoblasts through the pre-osteoblasts. The osteoblasts produce in a large amount extracellular matrices, such as collagen, a constituent of the bone in the maturation process, and also express an alkaline phosphatase to cause sedimentation of calcium phosphate to the matrices. A part of the osteoblasts is thus embedded in the calcified matrix, and further differentiated into osteocytes.

The amount of the bone in human shows its maximum at 20 to 30 years of age, and decreases thereafter. With aging, the number of osteoblasts in the osseous tissues decreases, and the differentiation ability from mesenchymal stem cells to osteoblasts is lowered. On the other hand, the number of fat cells derived from the mesenchymal stem cells similar to the osteoblasts is said to increase with aging. Therefore, it is considered to be effective from the viewpoint of prevention or treatment of osteoporosis that the amount of the osteoblasts and the osteocytes in the growth stage of the bone is increased, and that the osteogenesis is enhanced by more selectively promoting the differentiation from the mesenchymal stem cells to osteoblasts in the senile stage or post menopausal stage.

Recently, developments have been made on a drug for promoting osteogenesis, and there have been disclosed the action for promotion of osteogenesis by benzopyran derivatives (for instance, see JP-A-Hei-7-291983), phenyl-substituted hydroxycyclopentenone analogues (for instance, see JP-A-Hei-11-43460), prostaglandin A1 analogues (for instance, see
JP-A-Hei-11-43461), benzothiepin derivatives (for instance, see
JP-2000-109480 A), chromone derivatives (for instance, see JP-2001-139571 A). However, the evaluations for efficacy and safety are not yet satisfactory, so that these drugs have not reached a practical stage.

There has been found that a protein factor for inducing osteogenesis in the bone matrix, and named bone morphogenetic protein (hereinafter referred to as "BMP" in some cases). The entity thereof has been unknown for a long period of time. Taking the opportunity of cloning four kinds of BMP genes, it has been presently known that a dozen or so kinds of molecular species exist widely in animals beyond species. The BMP acts on pre-osteoblasts to increase alkaline phosphatase activity, responsiveness to parathyroid hormone, osteocalcine production, collagen synthesis or the like, thereby inducing the differentiation into osteoblasts. The BMP assigns the differentiation into cartilage cells, osteoblasts, or fat cells, depending upon the differentiation stage of the undifferentiated mesenchymal stem cells having multipotency. The BMP suppresses the differentiation into muscles of the myoblasts, and converts to the differentiation into osteoblasts. In addition, it has been known that the BMP has an inducing activity for a secondary growth factor such as insulin-like growth factor from osteoblasts. The osteogenesis is induced by administering the BMP together with a carrier subcutaneously or intramuscularly. Among the recombinant human BMPs, BMP-2, -4, -5, -6, and -7 are found to have activities for inducing osteogenesis alone. Especially, there has been known that recombinant human BMP-2 possesses a potent osteogenetic activity, and recovers defective tissues in bone defect model of a rat, sheep, a dog, a monkey and the like. In addition, there have been reported that BMP-4 and -5 are involved in the healing process of bone fracture, and that BMP-6 is involved in endochondral ossification. Also, there have been known that BMP-12 has an activity for forming ligaments and tendons, and that BMP-13 has an activity for forming cartilages. Further, there has been pointed out that the BMP is associated with senile osteoporosis because the lowering in the amount of the BMP in the bone matrix and the lowering in sensitivity of the osteoblasts to the BMP are found in aged animals or elderly human individuals.

The BMP plays an important role not only in osteogenesis but also in the developmental process. BMP-2, -4, -7, -8, -11 and the like are involved in formation of dorsabdominal axis or mesodermal formation, cardiogenesis, nephrogenesis, oculogenesis, spermatogenesis and the like. A knockout animal of BMP shows a lethal or severe disorder. As described above, the BMP is essential for a living body, and has been known to possess a variety of physiological activities.

Since the BMP shows various kinds of actions mentioned above, the BMP itself has been tried to be directly utilized as a protein preparation in the treatment of osteoporosis, bone fracture or the like. However, the BMP is a protein so that there arise problems in the limitations of the administration methods and occurrence of resistance. In addition, since the receptors for the BMP are widely expressed in numerous tissues, when the preparation is administered to a whole body, there is a risk that tissues other than the bones may be affected. Because of these drawbacks, the actual use of the BMP *per se* as a therapeutic agent is accompanied by various limitations. However, if the BMP production can be arbitrarily enhanced in a desired tissue site, not by an external administration, it is considered that the BMP is effective for treatment or prevention of a disease requiring enhancement of BMP production, such as osteoporosis or bone fracture. In recent years, there has been disclosed that a specified compound having two aromatic systems (see, for instance, WO 97/15308), or a statin compound such as mevastatin, lovastatin, pravastatin or simvastatin (see, for instance, WO 98/25460) possesses an enhancing activity for BMP-2 production. Also, there has been disclosed that helioxantin (see, for instance, JP-A-Hei-8-245386) or a condensed thiophene derivative (see, for instance, JP-A-Hei-9-151132) has an activity for enhancing the action of the BMP. However, these are still unsatisfactory in the evaluation of safety and efficacy, and they have not reached the stage of actual use.

In recent years, development has been made to utilize the BMP in osteoanagenetic medicine, and there has been tried to obtain a therapeutic effect by implanting a complex of the BMP and the carrier with an implanting material to a bone fracture site. However, since the BMP is brought into a living body in a large amount, there arise some problems from the aspects of safety and economic advantages. It is considered that the drawback can be avoided by the use of a substance for enhancing the BMP production or promoting the osteogenesis in place of administration of BMP, but a satisfactory means for actual use has not yet been known.

As described above, it is considered that by promoting the osteogenesis or enhancing the BMP production, various diseases associated therewith can be treated or prevented. However, substances, means and the like capable of appropriately enhancing the BMP production as desired without showing toxicity or adverse actions have not yet been known.

### DISCLOSURE OF INVENTION

An object of the present invention is to develop a composition having enhancing action for bone morphogenetic protein production or promoting action for osteogenesis suitable as food materials and medicament materials, which is capable of being conveniently taken, and to provide a medicament, food, beverage or feed using the composition.

Specifically, the present invention relates to:
[1] a therapeutic agent or prophylactic agent for a disease requiring enhancement of bone morphogenetic protein production or promotion of osteogenesis, characterized in that the therapeutic agent or prophylactic agent comprises as an effective ingredient at least one compound selected from the group consisting of (a) an acidic saccharide, (b) a polyacrylic acid, (c) chlorogenic acid and (d) an oxidation processed product of chlorogenic acid;
[2] the therapeutic agent or prophylactic agent according to the above [1], wherein the acidic saccharide is at least one acidic saccharide selected from the group consisting of an acidic polysaccharide, an acidic oligosaccharide, an acidic monosaccharide, and a degradation product thereof;
[3] the therapeutic agent or prophylactic agent according to the above [1], wherein the acidic saccharide is at least one acidic saccharide selected from the group consisting of fucoidan, heparin, a dextran sulfate, chondroitin sulfate B, pectic acid, an acidic polysaccharide derived from *Spirulina,* a fucoidan degradation product, carrageenan λ, carrageenan κ, carrageenan 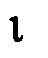 , a low-molecular weight heparin, a heparan sulfate and an acidic polysaccharide derived from *Chlorella;*
[4] an agent for enhancement of bone morphogenetic protein production or an agent for promotion of osteogenesis, characterized in that the agent comprises as an effective ingredient at least one compound selected from the group consisting of (a) an acidic saccharide, (b) a polyacrylic acid, (c) chlorogenic acid and (d) an oxidation processed product of chlorogenic acid;
[5] the agent for enhancement of bone morphogenetic protein production or the agent for promotion of osteogenesis according to the above [4], wherein the acidic saccharide is at least one acidic saccharide selected from the group consisting of an acidic polysaccharide, an acidic oligosaccharide, an acidic monosaccharide, and a degradation product thereof;
[6] the agent for enhancement of bone morphogenetic protein production or the agent for promotion of osteogenesis according to the above [4], wherein the acidic saccharide is at least one acidic saccharide selected from the group consisting of fucoidan, heparin, a dextran sulfate, chondroitin sulfate B, pectic acid, an acidic polysaccharide derived from *Spirulina,* a fucoidan degradation product, carrageenan λ, carrageenan κ, carrageenan 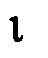 , a low-molecular weight heparin, a heparan sulfate and an acidic polysaccharide derived from *Chlorella;*
[7] a food, beverage or feed for enhancement of bone morphogenetic protein production or promotion of osteogenesis, characterized in that the food, beverage or feed comprises as an effective ingredient at least one compound selected from the group consisting of (a) an acidic saccharide, (b) a polyacrylic acid, (c) chlorogenic acid and (d) an oxidation processed product of chlorogenic acid;
[8] the food, beverage or feed for enhancement of bone morphogenetic protein production or promotion of osteogenesis according to the above [7], wherein the acidic saccharide is at least one acidic saccharide selected from the group consisting of an acidic polysaccharide, an acidic oligosaccharide, an acidic monosaccharide, and a degradation product thereof;
[9] the food, beverage or feed for enhancement of bone morphogenetic protein production or promotion of osteogenesis according to the above [7], wherein the acidic saccharide is at least one acidic saccharide selected from the group consisting of fucoidan, heparin, a dextran sulfate, chondroitin sulfate B, pectic acid, an acidic polysaccharide derived from *Spirulina,* a fucoidan degradation product, carrageenan λ, carrageenan κ, carrageenan 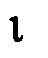 , a low-molecular weight heparin, a heparan sulfate and an acidic polysaccharide derived from *Chlorella;*
[10] a therapeutic agent or prophylactic agent for a disease requiring enhancement of bone morphogenetic protein production or promotion of osteogenesis, characterized in that the therapeutic agent or prophylactic agent comprises as an effective ingredient an extract derived from algae;
[11] an agent for enhancement of bone morphogenetic protein production or an agent for promotion of osteogenesis, characterized in that the agent comprises as an effective ingredient an extract derived from algae; and
[12] a food, beverage or feed for enhancement of bone morphogenetic protein production or promotion of osteogenesis, characterized in that the food, beverage or feed comprises as an effective ingredient an extract derived from algae.

### BRIEF DESCRIPTION OF THE DRAWINGS

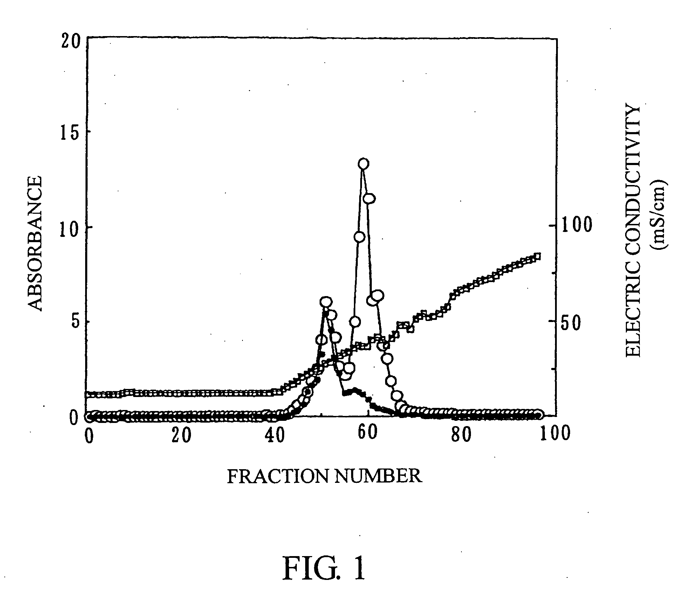

Figure 1 is a graph showing an elution pattern of the fucoidan derived from *Kjellmaniella crassifolia* on DEAE-Cellulofine A-800 column.

### BEST MODE FOR CARRYING OUT THE INVENTION

The terms "enhancing action for BMP production" and "enhancing activity for BMP production" as used herein each refers to provide enhancement of BMP production and a function for enhancing BMP production, and is not intended to particularly strictly distinguish in its meaning. In addition, the term "enhance" encompasses an embodiment in which the amount of the desired substance is increased after the action of the effective ingredient of the present invention as compared to that before the action, as well as an embodiment (induction) in which the desired substance is produced by the action of the effective ingredient of the present invention. In addition, any of the substances listed as the effective ingredient in the present specification can be used alone or in admixture of two or more kinds in the present invention.

As the effective ingredient usable in the present invention, at least one compound selected from the group consisting of (a) an acidic saccharide, (b) a polyacrylic acid, (c) chlorogenic acid and (d) an oxidation processed product of chlorogenic acid is used. The acidic saccharide is not particularly limited as long as the saccharide has an acidic group. For instance, at least one acidic saccharide selected from the group consisting of an acidic polysaccharide, an acidic oligosaccharide, an acidic monosaccharide, and a degradation product thereof can be used.

The acidic polysaccharide is not particularly limited as long as the saccharide has an enhancing action for BMP production or promoting action for osteogenesis. The acidic polysaccharide is exemplified by an acidic polysaccharide derived from algae, an acidic polysaccharide derived from Animalia, an acidic polysaccharide derived from Pisces, an acidic polysaccharide derived from Plantae, an acidic polysaccharide derived from microorganisms, and a synthetic acidic polysaccharide.

The acidic polysaccharides derived from algae include, for instance, sulfated polysaccharides derived from Phaeophyceae, such as sulfated fucose-containing polysaccharides such as fucoidan, sulfated fucogalactan (G-fucoidan), sulfated fucoglucuronomannan, glucuronoxylofucane, Sargassan, glucuronomannan galactan, xylofucoglucuronan, ascorfilan, glucuronogalactofucane, and sulfated glucuronofucane.

As the raw materials for those listed above, for instance, Phaeophyceae of Laminariales, Chordariales, Fucales, and the like, such as *Kjellmaniella crassifolia, Laminaria japonica, Kjellmaniella, Fucus, Nemacystus, Cladosiphon okamuranus, Undaria, Ecklonia kurome, Eisenia bicyclis, Ecklonia cava, Lessonia nigrescence* and *Ascophyllum nodosum* can be used.

In addition, as the acidic polysaccharide derived from algae, an acidic polysaccharide derived from Rhodophyceae, for instance, *Gelidium amansii, Gracilaria*, Giant kelp, or *Pteroclavia capillacae;* or carrageenan λ, carrageenan κ, or carrageenan 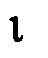 ; or the like can be used. Also, acidic polysaccharides derived from Cyanophyceae, for instance, acidic polysaccharides derived from *Spirulina;* and the acidic polysaccharides derived from Chlorophyceae, for instance, acidic polysaccharides derived from *Chlorella* can be used. Also, rhamnan sulfate derived from algae can be also used as the acidic polysaccharide of the present invention. In addition, phosphated polysaccharide, for instance, nucleic acid is encompassed by the acidic polysaccharide of the present invention.

The acidic polysaccharides used in the present invention are exemplified by, for instance, the fucoidans, which are sulfated fucose-containing polysaccharides mentioned above, and their derivations are not particularly limited, as long as the fucoidans are a polysaccharide having sulfated fucose as a constituent and have enhancing action for BMP production or promoting action for osteogenesis. There may be used fucoidans derived from Echinodermata, for instance, sea cucumber, Echnoidea, Asterozoa, and the like. Fucoidan-containing sea cucumber includes, for instance, sea cucumber disclosed in Japanese Patent Laid-Open No. Hei 4-91027, and the fucoidan can be prepared from sea cucumber by the method described in the publication.

For instance, a fucoidan is prepared from *Kjellmaniella crassifolia*, and the resulting fucoidan can be separated into glucuronic acid-containing fucoidan (referred to as "U-fucoidan") and glucuronic acid non-containing fucoidan (referred to as "F-fucoidan"), and each of the fucoidans can be used as the effective ingredient of the present invention. Also, sulfated fucogalactan can be prepared from *Kjellmaniella crassifolia* and used.

After the preparation of the fucoidans from *Kjellmaniella crassifolia*, U-fucoidan and F-fucoidan are separated by using an anionic exchange resin, a surfactant or the like. The existing ratio (weight ratio) of U-fucoidan to F-fucoidan derived from *Kjellmaniella crassifolia* is about 1:2. U-fucoidan contains fucose, mannose, galactose, glucuronic acid and the like, and its sulfate content is about 20% by weight. F-fucoidan contains fucose and galactose, and its sulfate content is about 50% by weight. The molecular weight for both substances is distributed, centering about 200000 (*Summary of 18th Sugar Symposium,* p. 159, 1996).

U-fucoidan and F-fucoidan can be separated, for instance, by applying a fucoidan solution prepared from *Kjellmaniella crassifolia* onto DEAE-Cellulofine A-800 column, and thereafter carrying out elution by the concentration gradient technique using NaCl-containing buffer. One of the examples is shown in Figure 1. Concretely, Figure 1 is a diagram showing the separation of U-fucoidan and F-fucoidan, wherein the former peak in the figure is U-fucoidan, and the latter peak is F-fucoidan.

Besides, as the fucoidans which are usable in the present invention, for instance, each of the fucoidan derived from *Fucus,* the fucoidan derived from *Nemacystus,* the fucoidan derived from *Cladosiphon okamuranus*, the fucoidan derived from *Undaria,* the fucoidan derived from *Lessonia,* the fucoidan derived from *Ascophyllum,* and the fucoidan derived from other algae can be prepared by a known method, and used in the present invention.

In addition, the acidic polysaccharide derived from Pisces includes, for instance, chondroitin sulfate derived from Chontropterygii such as *Chondrichthyes Selachii;* the acidic polysaccharide derived from Plantae includes, for instance, pectic acid, pectin or the like derived from apple, lemon, orange or the like; the acidic polysaccharide derived from microorganisms includes, for instance, colominic acid derived from *Escherichia coli,* teichuronic acid derived from *Bacillus subtilus,* and phosphomannan and phosphogalactan derived from yeasts; and the acidic polysaccharide derived from Animalia includes, for instance, heparin, low-molecular weight heparin, chondroitin sulfate, heparan sulfate, chondroitin, keratan sulfate, hyaluronic acid, and poly(ribose phosphate); and the like, respectively.

The synthetic acidic polysaccharide usable in the present invention is not particularly limited as long as the synthetic acidic polysaccharide has an enhancing action for BMP production or promoting action for osteogenesis, and the acidic polysaccharide which has been so far used as a medicament is suitably used. The synthetic acidic polysaccharide is exemplified by, for instance, synthetic sulfated polysaccharides such as sodium dextran sulfate. The compound is a sodium salt of sulfuric ester obtained by sulfating a partial degradation product of dextran produced by the fermentation of sucrose according to Leuconostoc mesenteroides van Tieghem.

In addition, as the synthetic sulfated polysaccharide in the present invention, for instance, sulfated products of dextran, dextran sulfate, dextrin, cyclodextrin, cellulose, starch, mannan, xylan, alginic acid, pectin, pectic acid, fructan, arabinan, chitin, pullulan, xyloglucan, starch, or the like can be used. Further, a synthetic sulfated polysaccharide such as ribofuranan sulfate, xylofuranan sulfate, lentinan sulfate, curdlan sulfate, or mannopyranan sulfate, or a synthetic sulfated alkyl polysaccharide such as ribofuranan sulfate having palmitoyl group can be used. The constitution of the alkyl group contained in the synthetic sulfated alkyl polysaccharide is not particularly limited, and the number of carbon atoms of the alkyl group may be usually 1 to 50 or so. A highly-sulfated sulfated polysaccharide or a highly-sulfated degradation product can be prepared by further sulfating the sulfated polysaccharide or a degradation product thereof. Each of these sulfated polysaccharides, highly-sulfated sulfated polysaccharides, and highly-sulfated degradation products may be prepared by a known method, and a degradation product thereof can also be prepared by a known method, and used in the present invention. Also, commercially available dextran sulfate and sulfated cellulose can be used, and salts of these synthetic sulfated polysaccharides and the like may be used.

When the sulfated polysaccharide is used as the acidic polysaccharide in the present invention, the sulfate content (or the number of sulfate groups) of the sulfated polysaccharide is not particularly limited, as long as the sulfated polysaccharide exhibits an enhancing action for BMP production or promoting action for osteogenesis. The degradation product of the acidic polysaccharide includes oligosaccharides and monosaccharides. For instance, fucose-2-sulfate or glucose-2-sulfate can be used. Each of these sulfated monosaccharides, sulfated oligosaccharides and sulfated polysaccharides may be prepared by a general synthesis method therefor, and the prepared product or purified product can be also used in the present invention. Here, in the present invention, the oligosaccharide is defined as a sugar compound in which the monosaccharides are connected with the range of from 2 to 10, and the polysaccharide is defined as a sugar compound in which 11 or more of the monosaccharides are connected.

In addition, the degradation products of the acidic polysaccharides, for instance, degradation products of sulfated polysaccharides and fucoidans, having enhancing action for BMP production or promoting action for osteogenesis can be prepared by a known method such as an enzymological method, a chemical method, or a physical method, and a desired degradation product having enhancing action for BMP production or promoting action for osteogenesis can be selected and used.

The degradation product refers to those obtained by degrading an acidic polysaccharide, wherein the degradation product has a molecular weight of preferably about 200 to about 100000, more preferably about 1000 to about 30000, depending upon the acidic polysaccharide to be degraded.

The preferable preparation method for the degradation product of the acidic polysaccharide used in the present invention is acid degradation method. By subjecting the acidic polysaccharide to an acid degradation, the degradation product having enhancing action for BMP production or promoting action for osteogenesis can be prepared.

The conditions for the acid degradation of the acidic polysaccharide used in the present invention are not particularly limited, as long as the conditions enable to generate the degradation product having promoting action for osteogenesis or enhancing action for BMP production (hereinafter referred to as "degradation product of the present invention").

For instance, the acidic polysaccharide is dissolved or suspended in an acidic aqueous solution or the like and subjected to the reaction, thereby generating a degradation product of the present invention. Also, the reaction mixture may be heated during the reaction, thereby shortening the time period required for the generation of the degradation product of the present invention.

The kinds of the acids for dissolving or suspending the acidic polysaccharide are not particularly limited. There can be used inorganic salts of hydrochloric acid, sulfuric acid, nitric acid and the like; organic acids such as citric acid, formic acid, acetic acid, lactic acid, malic acid and ascorbic acid; and solid acids such as cationic exchange resin, cationic exchange fiber and cationic exchange membrane.

The concentration of the acid is not particularly limited, and the acid can be used at a concentration of preferably from 0.0001 to 5 N or so, more preferably from 0.01 to 1 N or so. In addition, the reaction temperature is not particularly limited, and the reaction temperature may be set at preferably from 0° to 200°C, more preferably from 20° to 130°C.

In addition, the reaction time is not particularly limited, and the reaction time may be set at preferably from several seconds to several days. The kinds and the concentration of the acids, the reaction temperature, and the reaction time may be properly selected depending upon the generated amount of the degradation product used in the present invention and the degree of polymerization of the degradation product. For instance, during the preparation of the degradation product of the fucoidan, the organic acid such as citric acid, lactic acid or malic acid is used, and the concentration of the acid is properly selected from the range of several dozens mM to several M, the heating temperature from the range of 50° to 110°C, preferably 70° to 95°C, and the heating time from the range of several minutes to 24 hours, whereby the degradation product of the present invention can be prepared. The acid degradation product of the fucoidan is exemplified by the acid degradation product of the fucoidan derived from *Kjellmaniella crassifolia*, and this degradation product can be used as dietary fiber having enhancing action for BMP production or promoting action for osteogenesis.

The degradation product of the present invention can be fractionated by using its enhancing action for BMP production or promoting action for osteogenesis as an index. For instance, an acid degradation product can be fractionated based on a molecular weight by means of a fractionation method such as gel filtration method, molecular weight fractionation membrane, or the like.

As an example of gel filtration method, Cellulofine GCL-300 can be used to prepare any molecular weight fractions, for instance, one having a molecular weight exceeding 25000, one having a molecular weight of 25000 to exceeding 10000, one having a molecular weight of 10000 to exceeding 5000, one having a molecular weight of 5000 or less. Cellulofine GCL-25 can be used for the fraction having a molecular weight 5000 or less, to prepare any molecular weight fractions, for instance, one having a molecular weight of 5000 to exceeding 3000, one having a molecular weight of 3000 to exceeding 2000, one having a molecular weight of 2000 to exceeding 1000, one having a molecular weight of 1000 to exceeding 500, one having a molecular weight of 500 or less.

In addition, the molecular weight fractionation can be industrially carried out by using an ultrafiltration membrane. For instance, a fraction having a molecular weight of 30000 or less can be prepared by using FE10-FUSO382 manufactured by DAICEL CHEMICAL INDUSTRIES, LTD., and a fraction having a molecular weight of 6000 or less can be prepared by using FE-FUS-T653 manufactured by the same. Further, a fraction having a molecular weight of 500 or less can be obtained by using a nanofilter membrane. Any molecular weight fractions can be prepared by combining these gel filtration method and molecular weight fractionation method.

The degradation product of the acidic polysaccharide, for instance, the degradation product of the fucoidan, having the enhancing action for BMP production or promoting action for osteogenesis which can be used in the present invention is exemplified by the compound represented by the following formula (1): 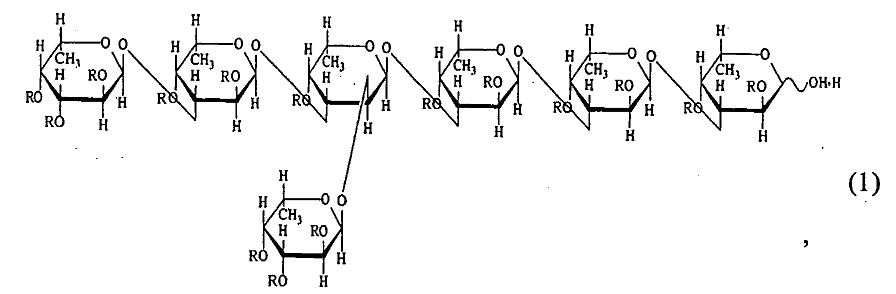 wherein R is H or SO₃H, wherein at least one ofR is SO₃H;
the compound represented by the following formula (2): 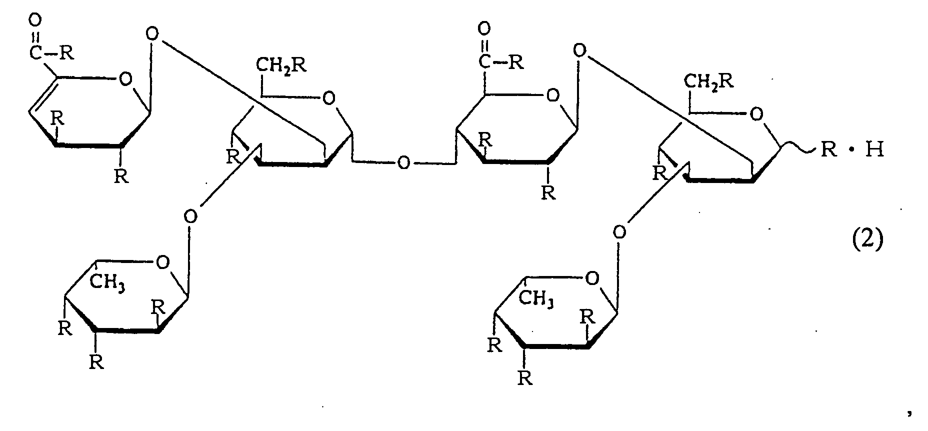 wherein R is OH or OSO₃H, wherein at least one of R is OSO₃H; the compound represented by the following formula (3): 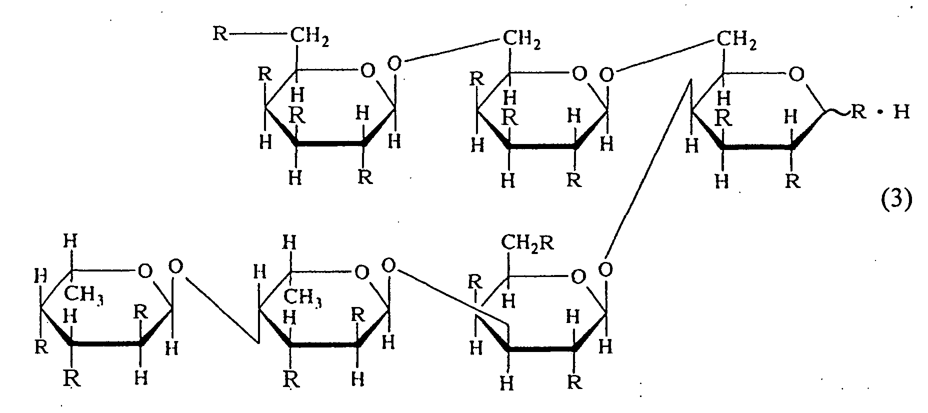 wherein R is OH or OSO₃H, wherein at least one of R is OSO₃H; and each of these compounds can be prepared in accordance with the method disclosed in Japanese Patent Laid-Open No. 2003-199596, WO 97/26896 and WO 00/50464. In the present specification, "•H" in the formula of the compound stands for a hydrogen atom at α-position or β-position bound to an anomeric carbon atom. The sulfated polysaccharides and oligosaccharides having a repeating structure of the compound represented by the formula (1), the formula (2) or the formula (3) can be also used as the sulfated saccharide having enhancing action for BMP production of the present invention.

In addition, the fucoidan derived from *Kjellmaniella crassifolia* is heat-treated in the presence of the organic acid, whereby a polymer of glucuronic acid and mannose can be obtained, and the resulting polymer can be also used as an acidic polysaccharide having enhancing action for BMP production or promoting action for osteogenesis. Also, by adjusting the heat treatment conditions and the heating time, polymers with any polymerization degrees can be prepared.

The acidic oligosaccharide preferably includes sulfated oligosaccharides, and the acidic monosaccharide preferably includes sulfated monosaccharides. As the sulfated oligosaccharide or sulfated monosaccharide, those commercially available can be used, or the sulfated oligosaccharide or sulfated monosaccharide can be prepared by sulfating each of various oligosaccharides or monosaccharides as a raw material by a known method. Also, salts thereof can be preferably used. Each of these can be used alone or in admixture of two or more kinds. In addition, the degradation product of the acidic oligosaccharide or acidic monosaccharide can be obtained in the same manner as in the degradation product of the acidic polysaccharide mentioned above. The acidic monosaccharide is not particularly limited, as long as the acidic monosaccharide having enhancing action for BMP production or promoting action for osteogenesis. The acidic monosaccharide is exemplified by, for instance, sulfated glucose, sulfated galactose, sulfated xylose, sulfated 2-deoxy-glucose, sulfated tallose and sulfated mannose. Further, the acidic polysaccharides, the acidic oligosaccharides and the acidic monosaccharides of the present invention encompass fatty acid-added derivatives of the sulfated polysaccharides, the sulfated oligosaccharides and the sulfated monosaccharides and the like.

The acidic saccharide usable in the present invention includes a salt thereof. The salt of the acidic saccharide usable in the present invention is exemplified by alkali metal salts, alkaline earth metal salts, salts with an organic base and the like. In addition, the salts include, for instance, salts with sodium, potassium, magnesium, or ammonium, or a salt with diethanolamine, ethylenediamine or the like. These salts can be obtained, for instance, by converting a sulfate group or a carboxyl group of the acidic saccharide usable in the present invention into a salt by a known method. The salt is preferably a pharmacologically acceptable salt.

In the present invention, as the acidic saccharide, at least one compound selected from the group consisting of acidic polysaccharides, acidic oligosaccharides, acidic monosaccharides and degradation products thereof is preferred, and more specifically at least one compound selected from the group consisting of fucoidan, heparin, a dextran sulfate, chondroitin sulfate (especially chondroitin sulfate B), pectic acid, an acidic polysaccharide derived from *Spirulina,* a fucoidan degradation product, carrageenan λ, carrageenan κ, carrageenan 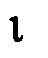 , a low-molecular weight heparin, a heparan sulfate, keratan sulfate, alginic acid, pectin, hyaluronic acid, and an acidic polysaccharide derived from *Chlorella* is preferably used. Among them, since any of the fucoidan, the compound represented by the above-mentioned formula (1) which is a degradation product thereof, the acidic polysaccharide derived from *Spirulina,* the acidic polysaccharide derived from *Chlorella* is obtained from marine algae which have been in our diet for a long period of time, these compounds are safe and have no concerns for side actions, and can be as a matter of course taken orally. Especially, the fucoidan derived from *Kjellmaniella crassifolia* can be most suitably used in the present invention because the fucoidan has a high enhancing activity for BMP-2 production and high safety.

The polyacrylic acid usable in the present invention is not particularly limited, and, for instance, those commercially available can be used. The molecular weight thereof is not particularly limited as long as the polyacrylic acid has an enhancing action for BMP production or promoting action for osteogenesis of the present invention. For instance, those having an average molecular weight of from 5000 to 1000000 can be used.

The chlorogenic acid usable in the present invention is not particularly limited, and, for instance, those commercially available, or those prepared from various plants in accordance with a known method can be used. The chlorogenic acid is a component contained in fruit or leaf of various Dicotyledonae plants such as plants belonging to Rubiaceae, Compositae, and Solanaceae. The chlorogenic acid prepared from any of the plants can be used in the present invention, as long as the chlorogenic acid has an enhancing action for BMP production or promoting action for osteogenesis of the present invention.

The oxidation processed product for the chlorogenic acid usable in the present invention is not particularly limited, and, for instance, a processed product obtained by subjecting the above-mentioned chlorogenic acid to an oxidation reaction can be used. The oxidation reaction is carried out, for instance, by subjecting chlorogenic acid to a process such as oxidation upon contact with oxygen, oxidation with an oxidizing agent, oxidation with an oxidation enzyme, or electric oxidation. Any of these oxidations can be carried out in accordance with known methods. For instance, when the oxidation is carried out upon the contact with oxygen, the oxidization can be carried out by feeding air through a pump to chlorogenic acid dissolved in an aqueous solution. The oxidization with an oxidizing agent can be carried out by preferably using as an oxidizing agent hydrogen peroxide, ozone, silver, copper, iron, nickel, manganese, cobalt, chromium or the like. The oxidation with an oxidation enzyme can be carried out by preferably using as an oxidation enzyme tyrosinase, peroxidase or the like. Incidentally, the oxidization with an oxidation enzyme also encompasses oxidation with a microorganism containing the oxidation enzyme exemplified above. In addition, when oxidation is carried out electrically, oxidation can be carried out by anodization reaction using as an electrode, for instance, platinum, lead oxide, carbon, graphite, platinum oxide or the like.

The structure of the composition obtained in accordance with the oxidation processing is unknown, and it is deduced that chlorogenic acid is simply oxidized, or polymerized to exist in the form of a polymer.

These effective ingredients can be used alone or in admixture of two or more kinds. These degradation products or salts of the acidic polysaccharides exemplified can be used without particular limitations as long as the degradation products or salts show an enhancing action for BMP production or promoting action for osteogenesis.

In the present invention, the extract derived from algae is not particularly limited, as long as the extract has an enhancing action for BMP production or promoting action for osteogenesis, and any of the algae can be used without particular limitation as long as the algae can be raw materials for previously mentioned acidic polysaccharides such as Phaephyceae, Cyanophyceae, Rhodophyceae and Chlorophyceae. Especially preferably, Phaephyceae such as *Kjellmaniella crassifolia, Chlorella* or *Spirulina* can be used. In the present invention, the extract refers to a substance obtained through the process of carrying out the extraction procedure with an extraction solvent. The extraction can be carried out as follows by a known extraction method. For instance, the raw material is powdered or cut into thin pieces, and thereafter extracted in a batch process or continuous process using a solvent. The extraction solvent used upon obtaining an extract is not particularly limited, and includes hydrophilic or lipophilic solvents such as water, chloroform, alcohols such as ethanol, methanol and isopropyl alcohol, ketones such as acetone and methyl ethyl ketone, methyl acetate, and ethyl acetate, which can be used alone or properly as a mixed solution as desired. In addition, the aqueous solution of the calcium salt can be preferably used as a solvent. The amount of the extraction solvent may be appropriately determined. Usually, the extraction solvent may be used in an amount of preferably from 0.1- to 100-folds that by weight of the raw materials subjected to the extraction procedure. The extraction temperature may be also appropriately determined according to its purposes. In the case of the water extraction, usually, the extraction temperature is in the range of preferably from 4° to 130°C, more preferably from 25° to 100°C. Alternatively, in the case where ethanol is contained in the solvent, the extraction temperature is suitably within the range of from 4° to 60°C. The extraction time may be also determined in consideration of extraction efficiency. It is usually preferable that the raw materials, the extraction solvent and the extraction temperature are set so that the extraction time is within the range of preferably from several seconds to several days, more preferably 5 minutes to 24 hours. The pressure during the extraction is not particularly limited, and can be properly determined as desired. The pressure may be, for instance, normal pressure, pressurized pressure, or reduced pressure by suction filtration or the like. The extraction procedure may be carried out, for instance, while stirring or allowing the mixture to stand. Also, the extraction procedures may be repeated several times as desired. By the above procedures, an extract derived from the algae (hereinafter which may be referred to as the extract of the present invention in some cases) is obtained. The extract is subjected to such a process as filtration, centrifugation, concentration, ultrafiltration or molecular sieving as desired, whereby an extract in which the component having an enhancing action for BMP production or promoting action for osteogenesis is concentrated can be prepared. In the present invention, the enhancing action for BMP production or promoting action for osteogenesis of the above-mentioned acidic polysaccharide, the extract or concentrated extract derived from algae to be used as the effective ingredient can be conveniently determined in accordance with the method described in Example 1 or 6 set forth below. In the present invention, two or more kinds of extracts obtained by different extraction methods can be contained to be used together.

In addition, in the present invention, a fraction obtained by fractionating an extract derived from algae by a known method, or a fraction obtained by repeating the fractionation procedures a plural times is also encompassed in the extract of the present invention. The above-mentioned fractionation means include extraction, separation by precipitation, column chromatography, thin-layer chromatography, and the like. The substance for enhancing BMP production or substance for promoting osteogenesis can also be isolated by further proceeding the purification of the resulting fraction using the enhancing action for BMP production or promoting action for osteogenesis as an index.

In the present invention, the shape of the extract derived from algae is not particularly limited as long as the extract has an enhancing action for BMP production or promoting action for osteogenesis, and the extract may take any form of powder, solid or liquid. In addition, the processed product of the extract having any given shape can be used as an extract derived from algae in the present invention in the form of a granular solid prepared by granulating the processed product by a known process. The granulation process is not particularly limited, and is exemplified by tumbling granulation, agitation granulation, fluidizing bed granulation, airflow granulation, extruding granulation, compression molding granulation, disintegration granulation, spray granulation, spray-drying granulation or the like. In addition, the extract in the form of powder can be dissolved in a liquid, for instance, water, an alcohol or the like to give a liquid form, and can be used as the extract of the present invention.

It is especially preferable that the extract in the present invention comprises a substance for enhancing BMP production or substance for promoting osteogenesis, for instance, an acidic polysaccharide, an acidic oligosaccharide, an acidic monosaccharide, or a degradation product thereof, in a high concentration and/or at high purity, as compared to that of the algae themselves. Here, the term "high concentration" means that the weight of the substance for enhancing BMP production or the substance for promoting osteogenesis per unit weight of the extract in the present invention is greater than that of the raw material algae. In addition, the term "high purity" means that the content ratio of the substance for enhancing BMP production or the substance for promoting osteogenesis in the extract is higher than that of the raw material algae.

No toxicity is especially found in the effective ingredient according to the present invention as mentioned later. Also, there is no risk of the onset of adverse actions. For these reasons, the disease can be safely and appropriately treated and prevented. Therefore, the therapeutic agent, the prophylactic agent, the food, the beverage or the feed of the present invention, each comprising the effective ingredient, is effective for treating or preventing a disease requiring enhancement for BMP production or promotion of osteogenesis.

In the present invention, BMP is exemplified by, for instance, BMP-1, BMP-2, BMP-3, BMP-4, BMP-5, BMP-6, BMP-7, BMP-8, BMP-9, BMP-10, BMP-11, BMP-12, BMP-13, BMP-14, BMP-15 and the like, and especially preferably BMP-2, BMP-4 or BMP-7 is exemplified. Also, the presence or absence of the enhancing action for BMP production is not particularly limited, and can be conveniently determined according to the method described in Example 1 set forth below.

The BMP is a factor for potently promoting the formation of bones, cartilages, ligaments, tendons and the like, and is thought to act on preosteoblasts to promote differentiation thereof into osteoblasts, to be involved in generation, growth, and remolding of the bone, and the healing process of the bone fracture. In addition, the BMP acts on undifferentiated mesenchymal stem cells to assign the differentiation into chondroblasts, osteoblasts or fat cells depending upon the differentiation stages, whereby greatly involved in the maturation and proliferation of mesenchymal stem cells. Furthermore, in the process of the development of an individual, the BMP plays a key role in formation of dorsabdominal axis, mesodermal formation or the like. Among the BMPs, BMP-2, -4, and -7 have especially potent bone morphogenetic activities, so that a recombinant human BMP-2 can act on a bone-defective animal to restore the bone defects.

BMP is a protein having high selectivity acting only on the cells having sensitivity. Further, the orientation of undifferentiated mesenchymal stem cells into osteoblasts by BMP has high specificity, so that the cells would not be differentiated into undesired cells other than osteoblasts. Therefore, there is no diseases, periodontium defects in periodontal diseases, tooth root or alveolar defects, ridge formation, and recovery of palatoschisis.

Since the therapeutic agent of the present invention can enhance BMP production or promote osteogenesis, the therapeutic agent can exhibit therapeutic effects for the diseases as mentioned above. In addition, the prophylactic agent of the present invention strengthens bones, cartilages or teeth by the action of the effective ingredient of the present invention, the prophylactic agent can exhibit the prophylactic effects for the diseases as mentioned above.

In addition, the therapeutic agent or prophylactic agent of the present invention can be also used as an osseous tissue restoration agent after surgery for multiple myeloma, lung cancer, breast cancer, and the like. Furthermore, the therapeutic agent or prophylactic agent of the present invention can be used for the osteoanagenesis purposes in the field of regenerative medicine. Concretely, the therapeutic agent or prophylactic agent of the present invention can be used for activating or stabilizing artificial bone or artificial tooth root. Also, cells can be taken from a living body of a patient before having the disease or a patient having the disease, and allowing the therapeutic agent or prophylactic agent of the present invention to act on the cells *in vitro* to form regenerated osseous tissues, and thereafter returning the tissues to the living body of the patient.

The therapeutic agent or prophylactic agent of the present invention includes one formed into a preparation by combining the above-mentioned effective ingredient according to the present invention with a known pharmaceutical carrier. The effective ingredient of the present invention can be also used together with, for instance, a drug for inhibiting the absorption of the bone, such as estrogen, calcitonin, activated vitamin D₃, bisphosphonate or the risk on the side actions for the effective ingredient of the present invention having an enhancing action for BMP production, thereby making it very useful as pharmaceuticals and functional foods.

The promoting action for osteogenesis in the present invention is not particularly limited, as long as a morphogenetic action of bones and cartilages is promoted. For instance, the promoting action for osteogenesis is exemplified by a promoting action for differentiation of mesenchymal stem cells into osteoblasts, a promoting action for differentiation of undifferentiated mesenchymal stem cells into osteoblasts, a promoting action for differentiation of preosteoblasts into osteoblasts, a promoting action for formation of bone matrix, an action of calcification of bone matrix, a promoting action for differentiation of osteoblasts into osteocytes, an inducing action for endochondral ossification, and the like. In addition, the presence or absence of the promoting action for osteogenesis is not particularly limited, and can be conveniently determined, for instance, as an action for promoting differentiation from mesenchymal stem cells into osteoblasts according to the method described in Example 6 set forth below. Here, the term "promoting" encompasses "inducing."

In the present invention, the disease requiring enhancement for BMP production or promotion of osteogenesis is exemplified, for instance, by osteoporosis (for instance, chronic osteoporosis, osteoporosis caused by abnormality of hormonal balance in postmenopausal stage, secondary osteoporosis accompanying diabetes or an adverse action of steroid or the like), bone fracture, bone refracture, bone defects, dysosteogenesis, osteomalacia, osteonecrosis ofBehçet's disease, ankylosing spondylitis, chronic articular rheumatism, osteoarthritis, osteoarthritis involving cartilages, periodontal like.

The therapeutic agent or prophylactic agent of the present invention is usually manufactured by formulating the above-mentioned effective ingredient with a pharmacologically acceptable liquid or solid carrier. A solvent, a dispersant, an emulsifier, a buffer, a stabilizer, an excipient, a binder, a disintegrant, a lubricant, or the like is added thereto as desired, so that a solid agent such as a tablet, a granule, a powder, a fine powder, and a capsule, or a liquid agent such as a common liquid agent, a suspension agent or an emulsion agent can be formed. In addition, there can be also made into a dry product which can be made liquid by adding an appropriate liquid carrier before use, or also into an external preparation.

The pharmaceutical carrier can be selected depending upon the administration form and preparation form of the therapeutic agent or prophylactic agent. In the case of an orally administered preparation comprising a solid composition, the preparation can be produced in the form of a tablet, a pill, a capsule, a powder, a fine powder, a granule or the like, and there can be utilized, for instance, starch, lactose, saccharose, mannitol, carboxymethyl cellulose, cornstarch, an inorganic salt or the like. In addition, during the preparation of the orally administered preparation, a binder, a disintegrant, a surfactant, a lubricant, a fluidity accelerator, a flavor, a colorant, a perfume, and the like can be further formulated. In the case of forming into a tablet or pill, for instance, the tablet or pill may be covered with a sugar-coating made of sucrose, gelatin or hydroxypropyl cellulose, or with a film made of a substance soluble in the stomach or intestine as desired. In the case of an orally administered preparation comprising a liquid composition, the preparation can be prepared in the form of a pharmaceutically acceptable emulsion, solution, suspension, syrup, or the like. In this case, for instance, purified water, ethanol or the like is utilized as a carrier. Furthermore, an auxiliary agent such as a wetting agent or a suspending agent; a sweetener, a flavor, an antiseptic, or the like may be added as desired.

On the other hand, in the case of a non-orally administered preparation, the preparation can be prepared by dissolving or suspending the above-mentioned effective ingredient of the present invention in a diluent such as distilled water for injection, physiological saline, an aqueous solution of glucose, vegetable oil for injection, sesame oil, peanut oil, soybean oil, corn oil, propylene glycol or polyethylene glycol, in accordance with a conventional method, and adding a microbicide, a stabilizer, an osmotic regulator, a soothing agent, or the like as desired. It is also possible to produce a solid composition which is dissolved in sterile water or a sterile solvent for injection before use.

The external preparation includes solid, semi-solid or liquid preparations for percutaneous administration or transmucosal (oral or intranasal) administration. The external preparation also includes suppositories and the like. For instance, the external preparation may be prepared as liquid preparations including emulsions, suspensions such as lotions, external tinctures, and liquid agents for transmucosal administration; ointments such as oily ointments and hydrophilic ointments; medical adhesives for percutaneous administration or transmucosal administration such as films, tapes and poultices; and the like.

Each of the above-mentioned various preparations can be appropriately produced in accordance with conventional methods by utilizing known pharmaceutical carriers and the like. Also, the content of the effective ingredient in the preparation is not particularly limited, as long as the content is in an amount so that the effective ingredient can be preferably administered within the dose described below in consideration of administration form, administration method and the like of the preparation. The content of the effective ingredient of the present invention is usually 0.0001 % by weight or more, preferably from 0.001 to 80% by weight, more preferably from 0.01 to 70% by weight, or so. Incidentally, in the present specification, when the extract of the present invention is used as the effective ingredient, its content in the medicament, the food, the beverage or the feed and the dose of the effective ingredient are all expressed as calculated on a dry weight basis of the extract of the present invention.

The therapeutic agent or prophylactic agent of the present invention is administered via an administration route appropriate for each of the preparation form. The administration method is also not limited to specific one. The agent can be administered internally, externally (or topically) or by injection. The injection can be administered, for instance, intravenously, intramuscularly, subcutaneously, intracutaneously, or the like. As to an external preparation, for instance, a suppository may be administered according to its proper administration method.

The dose of the therapeutic agent or prophylactic agent of the present invention is changeable and properly set depending upon its preparation form, administration method, purpose of use, and age, body weight, symptom or the like of a patient to which the therapeutic agent or prophylactic agent is applied, or the like. Generally, for instance, when a fucoidan derived from *Kjellmaniella crassifolia* is used as an effective ingredient, the dose of the agent, in terms of the dose of the above-mentioned effective ingredient contained in the preparation, is not particularly limited, and the dose is from 0.0001 µg to 2000 mg/kg body weight, preferably from 0.001 µg to 1000 mg/kg body weight, more preferably from 0.01 µg to 100 mg/kg body weight per day for human (for instance, adult). As a matter of course, the dose varies depending upon various conditions, so that an amount smaller than the dose mentioned above may be sufficient, or an amount exceeding the dose range may be required. Administration may be carried out once or in several divided portions in a day within the desired dose range. The administration period is not particularly limited. Also, the therapeutic agent or prophylactic agent of the present invention can be directly orally administered, or the agent can be added to any foodstuffs to be taken on a daily basis.

In addition, the present invention can provide an agent for enhancing BMP production or an agent for promoting osteogenesis, each comprising the above-mentioned effective ingredient. The agent for enhancing BMP production or the agent for promoting osteogenesis may be the above-mentioned effective ingredient itself, or a composition comprising the above-mentioned effective ingredient. The agent for enhancing BMP production or the agent for promoting osteogenesis may be prepared by, for instance, formulating the above-mentioned effective ingredient with other ingredients which can be used for the same application as the effective ingredient, and forming into a form of reagent usually used according to the above-mentioned process for preparing the therapeutic agent or prophylactic agent. The content of the above-mentioned effective ingredient in the agent for enhancing BMP production or the agent for promoting osteogenesis is not particularly limited, as long as the content is in an amount so that the desired effects of the present invention can be exhibited in consideration of administration method, purpose of use or the like of the agent for enhancing BMP production or the agent for promoting osteogenesis. For instance, the content of the effective ingredient of the present invention is usually from 0.01 to 100% by weight or so. Also, the amount of the agent for enhancing BMP production or the agent for promoting osteogenesis used is not particularly limited, as long as the desired effects of the present invention can be obtained. Especially in the case where the agent is administered to a living body, the agent may be preferably used in an amount so that the effective ingredient can be administered within the dose range of the effective ingredient for the above-mentioned therapeutic agent or prophylactic agent. The agent for enhancing BMP production or the agent for promoting osteogenesis is useful for a disease requiring enhancement for BMP production or promotion of osteogenesis.

In addition, the agent for enhancing BMP production or the agent for promoting osteogenesis can be used by containing in the implant. Therefore, by using in the implant, for instance, in bone fracture, the osteosynthesis can be promoted, whereby the healing of the bone fracture or integration of the implant and the osseous tissues can be accelerated. The term implant as used herein means an instrument at least partially introduced into a body during a surgery, and is used for breakage or damages of joints, bones, teeth, ligaments, tendons, or the like. Also, the implant may permanently remain in the body, and be reabsorbed in an organism. Here, the agent for enhancing BMP production or the agent for promoting osteogenesis may be contained in the internal of the implant, or may be contained by coating the implant surface with the agent. The content of the agent for enhancing BMP production or the agent for promoting osteogenesis of the present invention in the implant is not particularly limited, and is usually from 0.01 to 80% by weight or so.

In addition, the agent for enhancing BMP production or the agent for promoting osteogenesis of the present invention can be used to be contained in a dentifrice. The dentifrice can promote the re-calcification of teeth by the enhancing action for BMP production or the promoting action of osteogenesis of the present invention. The content of the agent for enhancing BMP production or the agent for promoting osteogenesis of the present invention in the dentifrice is not particularly limited, and is usually from 0.01 to 80% by weight or so.

In addition, the agent for enhancing BMP production or the agent for promoting osteogenesis is useful for screening of drugs for diseases involving bones. Furthermore, the agent for enhancing BMP production or the agent for promoting osteogenesis is useful for functional studies relating to physical changes of the bones.

In addition, the present invention provides a food, beverage or feed for enhancement of BMP production or promotion of osteogenesis, comprising the above-mentioned effective ingredient. The term "comprising(ed)" means containing(ed), adding(ed), and/or diluting(ed). Because of its enhancing action for BMP production or promoting action for osteogenesis, the food, beverage or feed of the present invention is very useful in amelioration of symptoms and prevention of a disease requiring enhancement of BMP production or promotion of osteogenesis. Therefore, the food or beverage of the present invention is very suitable for taking by one who is concerned about one's health of the bones, or one who is concerned about the bone density.

In the food, beverage or feed as used herein, the term "containing(ed)" refers to an embodiment of containing the effective ingredient usable in the present invention in the food, beverage or feed; the term "adding(ed)" refers to an embodiment of adding the effective ingredient usable in the present invention to a raw material for the food, beverage or feed; and the term "diluting(ed)" refers to an embodiment of adding a raw material for the food, beverage or feed to the effective ingredient usable in the present invention.

The process for preparing the food, beverage or feed of the present invention is not particularly limited. For instance, formulation, cooking, processing, and the like can be carried out in accordance with those employed for general foods, beverages or feeds, and the food, beverage or feed of the present invention can be prepared by the general process, as long as the resulting food, beverage or feed may contain the above-mentioned effective ingredient of the present invention, wherein the effective ingredient has enhancing action for BMP production or the promoting action of osteogenesis.

The food or beverage of the present invention is not particularly limited. The food or beverage includes, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham, sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chewing gums, candies, chocolates, biscuits, sweet bun, cake, rice cake snacks and rice snacks; alcohol beverages such as *sake,* Chinese liquor, wine, whiskey, Japanese distilled liquor (*shochu*)*,* vodka, brandy, gin, rum, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, binned or pouched foods such as rice topped cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste (*miso*) soup, precooked foods, precooked beverages, and precooked soup; frozen foods such as *sukiyaki,* pot-steamed hotchpotch, split and grilled eel, hamburger steak, *shao-mai*, dumpling stuffed with minced pork, various sticks, and fruit cocktails; solid foods; liquid foods (soups and the like); spices; and the like, in which each of the foods and beverages comprises the above-mentioned ingredient according to the present invention. In addition, as the food of the present invention, chewing gums, candies and the like are especially preferable. Since the food is chewed in the mouth for a given period of time, if the effective ingredient of the present invention is contained in the food, the effects exhibited by the effective ingredient of the present invention, for instance, regeneration effects of periodontal tissues or the promoting effects of re-calcification of teeth can be more effectively exhibited.

In the food or beverage of the present invention, its shape is not particularly limited, as long as the above-mentioned effective ingredient is contained, added and/or diluted, alone or in plurality, and the effective ingredient is contained in an amount necessary for exhibiting its enhancing action for BMP production or the promoting action of osteogenesis. For instance, the shape includes those which can be taken orally such as tablets, granules and capsules.

The content of the above-mentioned effective ingredient in the food or beverage of the present invention is not particularly limited, and the content can be appropriately selected from the viewpoints of sensory aspect and exhibition of the activity. For instance, when the fucoidan derived from *Kjellmaniella crassifolia* is used as the effective ingredient, the amount of the fucoidan taken is, but not particularly limited thereto, from 0.0001 % by weight or more, preferably from 0.001 to 50% by weight, more preferably from 0.006 to 10% by weight, per 100% by weight of the food, and the amount is 0.0001 % by weight or more, preferably from 0.001 to 50% by weight, more preferably from 0.006 to 10% by weight, per 100% by weight of the beverage. In addition, when the food or beverage of the present invention preferably comprises, for instance, the fucoidan derived from *Kjellmaniella crassifolia* as the effective ingredient, the amount of the fucoidan taken is from 0.0001 µg to 2000 mg/kg body weight, preferably from 0.001 µg to 1000 mg/kg body weight, more preferably from 0.01 µg to 100 mg/kg body weight, per day for human (for instance, adult). As a matter of course, the amount of taken varies depending upon various conditions, so that an amount smaller than the amount of taken mentioned above may be sufficient, or an amount exceeding the range of the amount of taken may be required.

In addition, the present invention provides a feed for an organism having the enhancing action for BMP production or the promoting action of osteogenesis, in which the above-mentioned effective ingredient is contained, added and/or diluted. In still another embodiment, the present invention also provides a method of feeding an organism, characterized by administering the above-mentioned effective ingredient to the organism. In still yet another embodiment, the present invention provides an organism feeding agent characterized in that the organism feeding agent comprises the above-mentioned effective ingredient.

In these inventions, the organisms are, for instance, culturing or breeding animals, pet animals, and the like. The culturing or breeding animal is exemplified by cattle, laboratory animals, poultry, pisces, crustaceae or shellfish. The feed is exemplified by a feed for sustenance of and/or amelioration in physical conditioning. The organism feeding agent is exemplified by immersion agents, feed additives, and beverage additives.

According to these inventions, the same effects can be expected to be exhibited as those of the above-mentioned therapeutic agent or prophylactic agent of the present invention, on the basis of the enhancing action for BMP production or the promoting action of osteogenesis of the above-mentioned effective ingredient usable in the present invention, in the organism exemplified above for applying these. In other words, a therapeutic or prophylactic effect for a disease requiring enhancement for bone morphogenetic protein production or promotion of osteogenesis can be exhibited in the organism.

For instance, when the fucoidan derived from *Kjellmaniella crassifolia* is used as an effective ingredient, the above-mentioned effective ingredient usable in the present invention is usually taken in an amount of preferably from 0.0001 µg to 2000 mg/kg body weight, preferably from 0.001 µg to 1000 mg/kg body weight, more preferably from 0.01 µg to 100 mg/kg body weight, per day for the subject organism. As a matter of course, the dose varies depending upon various conditions, for instance, the amounts of the solvents used in the case where the extract of the present invention is used, and the like, so that a dose smaller than the dose mentioned above may be sufficient, or a dose exceeding the range of the dose may be required. The administration can be made by previously adding and mixing the effective ingredient in a raw material for an artificially formulated feed to be given to a subject organism, or mixing the effective ingredient with a powder raw material for an artificially formulated feed, and thereafter further adding and mixing the mixture with other raw materials. The content of the above-mentioned effective ingredient in the feed is not particularly limited. The content can be appropriately set in accordance with its purposes. For instance, when the fucoidan derived from *Kjellmaniella crassifolia* is used as an effective ingredient, the content of the fucoidan is, but not particularly limited thereto, 0.0001% by weight or more, preferably from 0.001 to 50% by weight, more preferably from 0.006 to 10% by weight, of 100% by weight of the feed.

The process for preparing the feed according to the present invention is not particularly limited, and its composition may be set in accordance with a general feed, as long as the above-mentioned effective ingredient according to the present invention having the enhancing action for BMP production or the promoting action of osteogenesis may be contained in the feed prepared.

The organism to which the present invention can be applied is not limited. The culturing or breeding animals include cattle such as *Equus, Bos, Porcus, Ovis, Capra, Camelus,* and *Lama;* experimental animals such as mice, rats, guinea pigs, and rabbits; poultry such as *Chrysolophus*, ducks, *Meleagris,* and *Struthioniformes*; and the pet animals includes dogs, cats, and the like, so that the feed can be widely applied.

By allowing a subject organism to take the feed comprising the above-mentioned effective ingredient usable in the present invention having the enhancing action for BMP production or the promoting action of osteogenesis, or to immerse a subject organism into a solution containing the above-mentioned effective ingredient usable in the present invention having the enhancing action for BMP production or the promoting action of osteogenesis, the physical conditions of the cattle, experimental animals, poultry, pet animals or the like can be well sustained or ameliorated. These embodiments are one embodiment of the method of feeding an organism provided by the present invention.

No toxicity is found even when the above-mentioned effective ingredient usable in the present invention is administered to an organism in an amount effective for the exhibition of its action. For instance, in the case of oral administration, no cases of deaths are found even when the fucoidan derived from *Kjellmaniella crassifolia* is administered to a mouse at 1 g/kg in a single dose. In addition, no cases of deaths are found even when the above-mentioned effective ingredient is orally administered to a rat at 1 g/kg in a single dose.

### EXAMPLES

The present invention will be described more concretely hereinbelow by means of the examples, but the present invention is by no means limited to these descriptions. Unless specified otherwise, % in these examples means % by weight.

### Reference Example 1

(1) *Kjellmaniella crassifolia* was sufficiently dried, and thereafter 20 kg of the dried product was powdered with Jiyu Mill (manufactured by NARA MACHINERY CO., LTD.). In 900 liters of tap water was dissolved 7.3 kg of calcium chloride dihydrate (manufactured by Nippon Soda Co., Ltd.), and 20 kg of the powdered product of *Kjellmaniella crassifolia* was then mixed therewith. The resulting mixture was heated for 40 minutes until the liquid temperature was raised from 12° to 90°C by blowing steam. Thereafter, the mixture was kept at 90° to 95°C for 1 hour under stirring, and then cooled, to give 1100 liters of a cooled product. Next, the cooled product was subjected to solid-liquid separation with a solid-liquid separator (manufactured by West Farrier Separator, Model: CNA), to give about 900 liters of supernatant after solid-liquid separation. The amount 360 liters of the supernatant after solid-liquid separation was concentrated up to a volume of 20 liters with FE10-FC-FUS0382 (fraction molecular weight: 30000) manufactured by DAICEL CHEMICAL INDUSTRIES, LTD. Subsequently, the procedures of adding 20 liters of tap water and again concentrating the resulting liquid mixture up to a volume of 20 liters were repeated 5 times, and the concentrate was subjected to a desalting treatment, to give 25 liters of an extract derived from *Kjellmaniella crassifolia*. One liter of the extract was lyophilized, to give 13 g of a dried product of fucoidan derived from *Kjellmaniella crassifolia*.
(2) Seven grams of the dried product of fucoidan described in item (1) of Reference Example 1 was dissolved in 700 ml of a 20 mM imidazole buffer (pH 8.0) containing 50 mM sodium chloride and 10% ethanol, and insoluble matters were removed by centrifugation. The supernatant after centrifugation was applied onto a DEAE-Cellulofine A-800 column (φ 11.4 cm x 48 cm) equilibrated with the same buffer, and the column was then washed with the same buffer. The elution was carried out with a concentration gradient of from 50 mM to 1.95 M sodium chloride (250 ml per fraction). A total sugar content and an uronic acid content were determined by the phenol-sulfuric acid method and the carbazole-sulfuric acid method, to give Fractions 43 to 49, Fractions 50 to 55, and Fractions 56 to 67, in the order of elution. Next, these fractions were desalted by electrodialysis, and thereafter lyophilized, to give each of Fraction I (340 mg) from Fractions 43 to 49, Fraction II (870 mg) from Fractions 50 to 55, and Fraction III (2.64 g) from Fractions 56 to 67. Figure 1 shows an elution pattern of the fucoidan derived from *Kjellmaniella crassifolia* on the DEAE-Cellulofine A-800 column. In Figure 1, the axis of ordinates is the absorbance at 530 nm as determined by the carbazole-sulfuric acid method (solid circles in the figure), the absorbance at 480 nm as determined by the phenol-sulfuric acid method (open circles in the figure), and the electric conductivity (mS/cm: open squares in the figure), and the axis of abscissas is the fraction number.

### Reference Example 2

(1) A 2-liter Erlenmeyer flask was charged with 600 ml of a culture medium comprising an artificial sea water (manufactured by Jamarin Laboratory), pH 8.2, containing 0.25% glucose, 1.0% peptone, and 0.05% yeast extract, and then sterilized (at 120°C for 20 minutes). *Alteromonas* sp. SN-1009 (CCRC910070) was inoculated into the culture medium, and cultured at 25°C for 26 hours, to give a seed culture medium. A 30-liter jar fermentor was charged with 20 liters of a culture medium comprising an artificial sea water, pH 8.0, containing 1.0% peptone, 0.02% yeast extract, 0.2% sulfated polysaccharide described in item (2) of Reference Example 2 described below, and 0.01 % defoaming agent (manufactured by Shin-Etsu Chemical Co., Ltd., KM70), and sterilized at 120°C for 20 minutes. After cooling, 600 ml of the above-mentioned seed culture medium was inoculated, and cultured at 24°C for 24 hours under the conditions of 10 liters of aeration per minute and a stirring rate of 250 rpm. After termination of the culture, the culture medium was centrifuged, to give cells and culture supernatant. The culture supernatant obtained was concentrated with an ultrafilter equipped with holofiber having an excluding molecular weight of 10000, and the concentrate was then subjected to salting out with an 85% saturated ammonium sulfate. Precipitates formed were collected by centrifugation, and sufficiently dialyzed against a 20 mM Tris-HCl buffer (pH 8.2) containing an artificial sea water at a one-tenth concentration, to give 600 ml of a solution of an endo-sulfated polysaccharide-degrading enzyme, selectively acting on the sulfated polysaccharide.
(2) Two kilograms of dried *Kjellmaniella crassifolia* was powdered with a cutter mill (manufactured by MASUKO SANGYO CO., LTD.) fitted with a screen having a diameter of 1 mm, and the resulting seaweed chips were suspended in 20 liters of 80% ethanol. The suspension was stirred at 25°C for 3 hours and filtered with a filter paper, and thereafter the residue was sufficiently washed. The residue obtained was suspended in 40 liters of a 20 mM sodium phosphate buffer, pH 6.5, which was heated to 95°C, containing 50 mM sodium chloride. The suspension was treated at 95°C for 2 hours with occasional stirring, to extract a sulfated polysaccharide. The suspension of the extract was filtered, to give a filtrate. Thereafter, the filtration residue was washed with 3.5 liters of 100 mM sodium chloride, to give an additional filtrate. Both filtrates were combined, and then the temperature was lowered to 30°C. After 3000 U of alginic acid lyase K (manufactured by Nagase Biochemicals, Ltd.) was added to the resulting mixture, 4 liters of ethanol was added thereto. The resulting mixture was stirred at 25°C for 24 hours. Next, the mixture was centrifuged, and the resulting supernatant was concentrated up to a volume of 4 liters with an ultrafilter equipped with holofiber having an excluding molecular weight of 100000. Further, the ultrafiltration was continued with 100 mM sodium chloride containing 10% ethanol until a colored substance was no longer filtered. Precipitates formed in a non-filtrate solution were removed by centrifugation, and the temperature of the resulting supernatant was lowered to 5°C. The pH was adjusted to 2.0 with 0.5 N hydrochloric acid, and thereafter the formed precipitates such as a protein were removed by centrifugation. The pH of the resulting supernatant was immediately adjusted to 8.0 with 1 N sodium hydroxide. Next, an ultrafiltration was carried out with an ultrafilter equipped with holofiber having an excluding molecular weight of 100000, and the solvent was completely substituted with 20 mM sodium chloride, pH 8.0. Thereafter, the pH was again adjusted to 8.0, and the resulting mixture was centrifuged and then lyophilized, to give about 95 g of a sulfated polysaccharide.
(3) Two kilograms of dried *Kjellmaniella crassifolia* was powdered with a cutter mill fitted with a screen having a diameter of 1 mm, and the resulting seaweed chips were suspended in 20 liters of 80% ethanol. The suspension was stirred at 25°C for 3 hours, and filtered with a filter paper. Thereafter, the residue was sufficiently washed. The residue obtained was suspended in 20 liters of a buffer (pH 8.2) containing 30 ml of the solution of the endo-sulfated polysaccharide-degrading enzyme prepared in the above-mentioned item (1) of Reference Example 2, 10% ethanol, 100 mM sodium chloride, 50 mM calcium chloride and 50 mM imidazole, and the resulting mixture was stirred at 25°C for 48 hours. This suspension was filtered with a stainless screen having a screen-opening diameter of 32 µm, and the residue was washed with 10% ethanol containing 50 mM calcium chloride. Further, the residue was suspended in 10 liters of 10% ethanol containing 50 mM calcium chloride, and the suspension was stirred for 3 hours. Thereafter, the suspension was filtered with the stainless screen, and the residue was washed. Further, the residue was suspended under the same conditions, and the suspension was then stirred for 16 hours. The suspension was filtered with a stainless screen having a diameter of 32 µm, and the residue was washed. The filtrate and the washings thus obtained were collected, and the combined mixture was subjected to ultrafiltration with an ultrafilter equipped with holofiber having an excluding molecular weight of 3000, thereby separating a filtered solution from a non-filtered solution. This filtered solution was concentrated to a volume of about 3 liters with a rotary evaporator, and thereafter the concentrate was centrifuged, to give supernatant. The supernatant obtained was desalted with an electric dialyzer equipped with a membrane having an excluding molecular weight of 300. To the resulting solution was added calcium acetate so as to give a concentration of 0.1 M, and precipitates formed were removed by centrifugation. The resulting supernatant was applied onto a DEAE-Cellulofine column (amount of resin: 4 liters) previously equilibrated with 50 mM calcium acetate, and sufficiently washed with 50 mM calcium acetate and 50 mM sodium chloride. Thereafter, the elution was carried out with a gradient of from 50 mM to 800 mM sodium chloride. The amount collected at this time was 500 ml per fraction. The collected fraction was analyzed by cellulose acetate membrane electrophoresis [*Analytical Biochemistry,* **37,** 197-202 (1970)]. As a result, a sulfated saccharide which was eluted on a concentration of about 0.4 M sodium chloride (Proximity of Fraction No. 63) was homogeneous. Then, a solution of Fraction No. 63 was first concentrated to a volume of 150 ml, and thereafter sodium chloride was added so as to give a concentration of 4 M. The resulting solution was applied onto a Phenyl-Cellulofine column (amount of resin: 200 ml) previously equilibrated with 4 M sodium chloride, and sufficiently washed with 4 M sodium chloride. Non-adsorptive sulfated saccharide fractions were collected, and desalted with an electrodialyzer equipped with a membrane having an excluding molecular weight of 300, to give 505 ml of a desalted solution. Forty milliliters of the desalted solution obtained was applied onto a Cellulofine GCL-90 column (4.1 cm x 87 cm) equilibrated with 0.2 M sodium chloride containing 10% ethanol, to perform gel filtration. The collection was performed at 9.2 ml per fraction. All of the fractions were analyzed for a total sugar content by the phenol-sulfuric acid method [*Analytical Chemistry,* **28,** 350 (1956)]. As a result, since the sulfated saccharide formed a single peak, Fraction Nos. 63 to 70, which were fractions corresponding to a central part of the peak were collected. The combined fraction was desalted with an electrodialyzer equipped with a membrane having an excluding molecular weight of 300, and thereafter 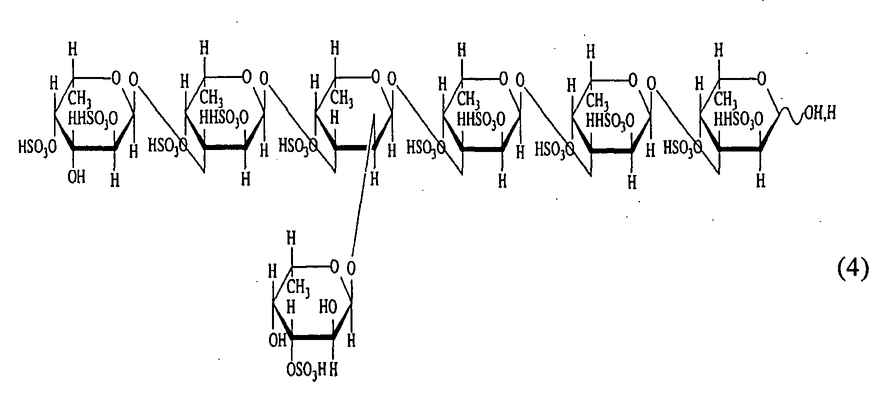 lyophilized, to give 112 mg of a dried product of the compound represented by the following formula (4). The compound is hereinafter referred to as 7-12SFd-F.

### Reference Example 3

Twenty grams of dry spirulina powder (sold by Spirulina Bio-Lab Co., Ltd.) was placed in a homogenizer (manufactured by Nippon Seiki Co., Ltd.), and 400 ml of acetone was added thereto. The mixture was homogenized at 8000 rpm for 10 minutes. The homogenate was filtered with a filter paper, to give a residue. The procedure of washing the residue with acetone was repeated 3 times in the same manner as the above, to give an acetone-washed residue. The acetone-washed residue was washed 4 times with 90% ethanol and 4 times with 80% ethanol in the same manner as the washing with acetone, to give an ethanol-washed residue. To the ethanol-washed residue was added 600 ml of 30 mM phosphate buffer (pH 7.0) containing 100 mM sodium chloride and 10% ethanol, and the mixture was stirred at room temperature for 18 hours. This mixture was centrifuged at 10000 rpm for 40 minutes, to give a supernatant. The insoluble substances contained in the supernatant were filtered with a filter paper, to give a crude extract (filtrate). The resulting crude extract was concentrated to a volume of 300 ml with an ultrafilter equipped with hollow fiber having an excluding molecular weight of 10000, and the concentrate was then subjected to ultrafiltration with adding 2 liters of 100 mM sodium chloride containing 10% ethanol thereto. Thereafter, the solvent was exchanged with 10 mM imidazole-hydrochloric acid buffer (pH 7.0) containing 10% ethanol and 50 mM sodium chloride, to give 240 ml of *Spirulina* macromolecular fraction.

The *Spirulina* macromolecular fraction was applied onto a DEAE-Cellulofine A-800 column (Φ 3 × 14.2 cm) equilibrated with 10 mM imidazole-hydrochloric acid buffer (pH 7.0) containing 10% ethanol and 50 mM sodium chloride. The column was washed with 360 ml of the same buffer, and elution was then carried out with a gradient of from 0.05 M (200 ml) to 2 M (200 ml) sodium chloride. The eluate was fractionated in an amount of 10 ml per fraction. Of the eluted fractions, Fraction Nos. 14 to 30 were named *Spirulina* acidic polysaccharide fraction-I (SSP-I), Fraction Nos. 69 to 77 *Spirulina* acidic polysaccharide fraction-II (SSP-II), Fraction Nos. 78 to 83 *Spirulina* acidic polysaccharide fraction-III (SSP-III), and Fraction Nos. 84 to 99 *Spirulina* acidic polysaccharide fraction-IV (SSP-IV), respectively. SSP-I, SSP-II, SSP-III and SSP-IV were sufficiently dialyzed against distilled water and lyophilized, thereby consequently giving 200 mg, 260 mg, 100 mg and 60 mg, respectively.

### Reference Example 4

Twenty grams of dry *Chlorella* powder was placed in a homogenizer (manufactured by Nippon Seiki Co., Ltd.), and 400 ml of acetone was added thereto. The mixture was homogenized at 8000 rpm for 10 minutes. The homogenate was filtered with a filter paper, to give a residue. The procedure of washing the residue with acetone was repeated 3 times in the same manner as the above, to give an acetone-washed residue. The acetone-washed residue was washed 4 times with 90% ethanol and 4 times with 80% ethanol in the same manner as the washing with acetone, to give an ethanol-washed residue.

To the ethanol-washed residue was added 600 ml of 30 mM phosphate buffer (pH 7.0) containing 100 mM sodium chloride and 10% ethanol, and the mixture was stirred at room temperature for 18 hours. The mixture was centrifuged at 10000 rpm for 40 minutes, to give a supernatant. The insoluble substances contained in the supernatant were filtered with a filter paper, to give a crude extract (filtrate). The resulting crude extract was concentrated to a volume of 310 ml with an ultrafilter equipped with hollow fiber having an excluding molecular weight of 10000, and the concentrate was then subjected to ultrafiltration with adding 3 liters of 100 mM sodium chloride containing 10% ethanol. Thereafter, the solvent was exchanged with 10 mM imidazole-hydrochloric acid buffer (pH 7.0) containing 10% ethanol and 50 mM sodium chloride, to give 203 ml of *Chlorella* macromolecular fraction.

The *Chlorella* macromolecular fraction was applied onto a DEAE-Cellulofine A-800 column (Φ 3 × 14.2 cm) equilibrated with 10 mM imidazole-hydrochloric acid buffer (pH 7.0) containing 10% ethanol and 50 mM sodium chloride. The column was washed with 297 ml of the same buffer, and elution was then carried out with a gradient of from 0.05 M (200 ml) to 2 M (200 ml) sodium chloride. The eluate was fractionated in an amount of 10 ml per fraction. Of the eluted fractions, Fraction Nos. 63 to 68 were named *Chlorella* sulfated polysaccharide fraction-I (CPS-I), and Fraction Nos. 69 to 75 *Chlorella* sulfated polysaccharide fraction-II (CPS-II), respectively. CSP-I and CSP-II were sufficiently dialyzed against distilled water, and lyophilized thereby consequently giving 140 mg and 200 mg, respectively.

### Reference Example 5

Chlorogenic acid was dissolved in 100 mM sodium carbonate buffer (pH 9) so as to have a concentration of 100 mM. Air was blown into the solution via a Perister pump for 12 hours, thereby giving an oxidation processed product of chlorogenic acid.

### Example 1

Human osteosarcoma cell strain HuO9 was suspended in a DMEM medium (manufactured by Bio Whittaker) containing 10% fetal bovine serum (manufactured by Bio Whittaker) so as to have a concentration of 1 × 10⁵ cells/ml. The suspension was put into a 96-well plate in an amount of 0.1 ml per well, and the cells were cultured sterilely. After the cells were cultured for 2 days, the medium was exchanged with a fresh medium. The fucoidan derived from *Kjellmaniella crassifolia* obtained in item (1) of Reference Example 1 was added thereto as a sample, and the cells were cultured for 48 hours. Next, the concentration of bone morphogenetic protein-2 (BMP-2) in the culture medium was measured with enzyme immunoassay method (BMP-2 Immunoassay: manufactured by GT). The enhancing activity for BMP-2 production was expressed by setting as a control one without addition of the sample, and defining the BMP-2 concentration in the cell culture medium (the amount of BMP-2 production of a cell) as 100%. The amount of the sample was as shown in Table 1. As a result, it was revealed that the fucoidan derived from *Kjellmaniella crassifolia* obtained in item (1) of Reference Example 1 enhances BMP-2 production in a concentration-dependent manner. The results are shown in Table 1.

**Table 1**

| Enhancing Action for BMP-2 Production of Fucoidan Derived from *Kjellmaniella crassifolia* | |
|---|---|
| Amount (µg/ml) | Enhancing Activity for BMP-2 Production (%) |
| 0 | 100 |
| 500 | 2203.0 |
| 1000 | 2259.6 |
| Here, the amount of BMP-2 production of the control was 0.120 ng/ml. | |

### Example 2

The enhancing activities for BMP-2 production of the fucoidan derived from *Kjellmaniella crassifolia* prepared in item (1) of Reference Example 1, the fucoidan Fraction I, the fucoidan Fraction II and the fucoidan Fraction III prepared in item (2) of Reference Example 1, 7-12SFd-F prepared in item (3) of Reference Example 2, spirulina acidic polysaccharide fractions SSP-I, SSP-III, and SSP-IV prepared in Reference Example 3, and heparin (manufactured by Wako Pure Chemical Industries, Ltd.), dextran sulfate (manufactured by Sigma), chondroitin sulfate B (manufactured by Seikagaku Corporation) and pectic acid (manufactured by Nakalai Tesque, Inc.) were evaluated in the same manner as in Example 1. The amount of the sample was as shown in Table 2 or 3. As a result, it was revealed that the fucoidan derived from *Kjellmaniella crassifolia*, the fucoidan Fraction I, fucoidan Fraction II and fucoidan Fraction III, 7-12SFd-F, spirulina acidic polysaccharide fractions SSP-I, SSP-III, and SSP-IV, heparin, dextran sulfate, chondroitin sulfate B and pectic acid enhance BMP-2 production in a concentration-dependent manner. The results are shown in Tables 2 and 3.

**Table 2**

| Name of Sample | Amount | Enhancing Activity for BMP-2 Production |
|---|---|---|
| | (µg/ml) | (%) |
| Fucoidan Derived from *Kjellmaniella crassifolia* | 10 | 1674 |
| | 100 | 2072 |
| Fucoidan Fraction I | 10 | 1104 |
| | 100 | 1391 |
| Fucoidan Fraction II | 10 | 1341 |
| | 100 | 1578 |
| Fucoidan Fraction III | 10 | 1064 |
| | 100 | 1596 |
| SSP-I | 10 | 378.9 |
| | 100 | 1811 |
| SSP-III | 100 | 504.4 |
| SSP-IV | 10 | 1257 |
| | 100 | 1779 |
| Heparin | 10 | 435.1 |
| | 100 | 1339 |
| Dextran Sulfate | 10 | 2789 |
| | 100 | 2984 |
| Here, the amount of BMP-2 production for the control of the fucoidan derived from *Kjellmaniella crassifolia*, fucoidan Fraction I, fucoidan Fraction II and fucoidan Fraction III, SSP-I, III, IV, heparin and dextran sulfate was 0.110 ng/ml. | | |

**Table 3**

| Name of Sample | Amount | Enhancing Activity for BMP-2 Production |
|---|---|---|
| | (µg/ml) | (%) |
| 7-12SFd-F | 500 | 302.5 |
| | 1000 | 620.6 |
| Chondroitin Sulfate B | 500 | 524.5 |
| | 1000 | 846.2 |
| Pectic Acid | 500 | 220.3 |
| | 1000 | 242.1 |
| Here, the amount of BMP-2 production of the control of 7-12SFd-F was 0.201 ng/ml, and the amount of BMP-2 production of the control of chondroitin sulfate B and pectic acid was 0.130 ng/ml. | | |

### Example 3

The enhancing activities for BMP-2 production of carrageenan λ (manufactured by Sigma), carrageenan κ (manufactured by Sigma), carrageenan 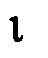 (manufactured by Sigma) and low-molecular-weight heparin (manufactured by CELSUS LABORATORIES) were evaluated in the same manner as in Example 1. The amount of the sample was as shown in Table 4. As a result, it was revealed that carrageenan λ, carrageenan κ, carrageenan 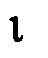 and low-molecular-weight heparin enhance BMP-2 production in a concentration-dependent manner. The results are shown in Table 4.

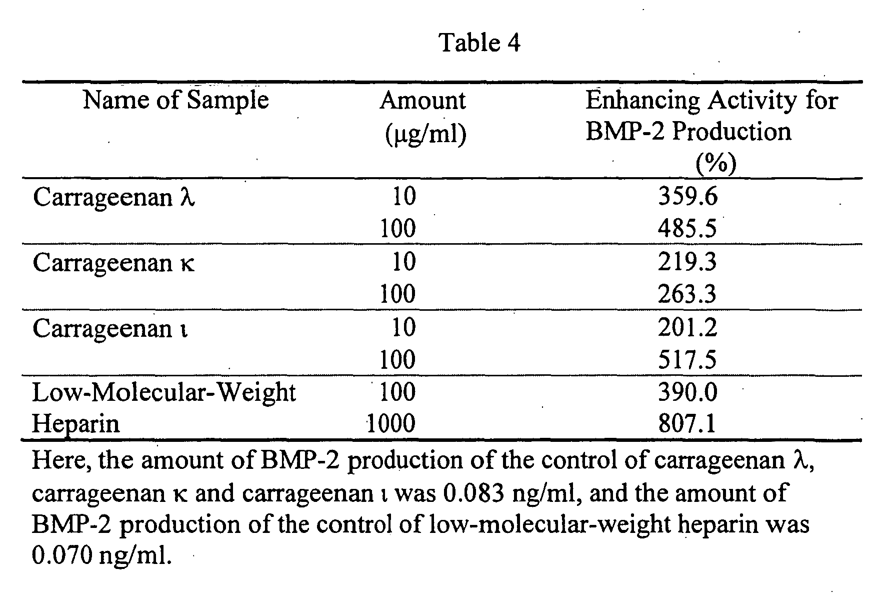

### Example 4

The enhancing activities for BMP-2 production of polyacrylic acids (average molecular weights: 5000, 250000 and 1000000: manufactured by Wako Pure Chemical Industries, Ltd.) were evaluated in the same manner as in Example 1. The amount of the sample was as shown in Table 5. As a result, it was revealed that the polyacrylic acids (average molecular weights: 5000, 250000 and 1000000) enhance BMP-2 production in a concentration-dependent manner. The results are shown in Table 5.

**Table 5**

| Name of Sample | Amount (µg/ml) | Enhancing Activity for BMP-2 Production (%) |
|---|---|---|
| Polyacrylic acid (Average Molecular Weight: 5000) | 10 | 191.3 |
| | 100 | 321.7 |
| Polyacrylic acid (Average Molecular Weight: 250000) | 10 | 310.9 |
| | 100 | 426.1 |
| Polyacrylic acid (Average Molecular Weight: 1000000) | 10 | 328.3 |
| | 100 | 371.7 |
| Here, the amount of BMP-2 production of the control was 0.046 ng/ml. | | |

### Example 5

The enhancing activities for BMP-2 production of chlorogenic acid (manufactured by Tokyo Kasei Kogyo Co., Ltd.) and the oxidation processed products of chlorogenic acid prepared in Reference Example 5 were evaluated in the same manner as in Example 1. The amount of the sample was as shown in Table 6. As a result, it was revealed that chlorogenic acid and the oxidation processed products of chlorogenic acid enhance BMP-2 production in a concentration-dependent manner. The results are shown in Table 6.

**Table 6**

| Name of Sample | Amount | Enhancing Activity for BMP-2 Production |
|---|---|---|
| | (µg/ml) | (%) |
| Chlorogenic Acid | 88.6 | 319.5 |
| | 177.2 | 320.4 |
| Oxidization Processed | 88.6 | 612.2 |
| Product of Chlorogenic Acid | 177.2 | 839.0 |
| Here, the amount of BMP-2 production of the control was 0.041 ng/ml. | | |

### Example 6

Mouse embryo derived cell strain C3H10T1/2 was suspended in a DMEM medium containing 10% fetal bovine serum so as to have a concentration of 3 × 10⁴ cells/ml. The suspension was put into a 96-well plate in an amount of 0.1 ml per well, and the cells were cultured sterilely. After the cells were cultured for 3 days, the medium was exchanged with a fresh medium. Heparan sulfate, or the *Chlorella* sulfated polysaccharide fraction CSP-I or CPS-II obtained in Reference Example 4 was added thereto as a sample, and the cells were cultured for 7 days. Subsequently, differentiation of C3H10T1/2 into osteoblasts was determined using an increase of the alkaline phosphatase activity in the cells as an index. The cells were washed once with PBS. One-hundred microliters of a reaction substrate solution (100 mM diethanolamine buffer pH 10.0, 2 mM magnesium chloride, 1 mM p-nitrophenyl phosphoric acid) was added thereto, and the reaction was carried out at 37°C for 30 minutes. Next, 100 µl of 0.2 N sodium hydroxide was added thereto to stop the reaction, and the amount of p-nitrophenol which was released was determined with an absorbance at 405 nm. The alkaline phosphatase activity when a sample was added was expressed by setting as a control one without addition of the sample, wherein the alkaline phosphatase activity of the control was defined as 100%. The activity indicates an induction activity of differentiation into osteoblasts. The amount of the sample was as shown in Table 7. The experiments were carried out twice, and the average value was taken. As a result, it was revealed that heparan sulfate, CSP-I and CSP-II induce differentiation into osteoblasts in a concentration-dependent manner. The results are shown in Table 7.

**Table 7**

| Name of Sample | Amount (µg/ml) | Alkaline Phosphatase Activity (%) |
|---|---|---|
| Heparan Sulfate | 10 | 132.2 |
| | 100 | 178.0 |
| CSP-I | 10 | 154.2 |
| | 100 | 228.0 |
| CSP-II | 10 | 113.6 |
| | 100 | 164.4 |

### INDUSTRIAL APPLICABILITY

According to the present invention, a medicament for treatment or prevention for a disease requiring enhancement of BMP production or promotion of osteogenesis, an agent for enhancement of BMP production or an agent for promotion of osteogenesis, a food, beverage or feed for enhancement of BMP production or promotion of osteogenesis are provided, wherein each comprises as an effective ingredient at least one compound selected from the group consisting of (a) an acidic saccharide, (b) a polyacrylic acid, (c) chlorogenic acid and (d) an oxidation processed product of chlorogenic acid, or an extract derived from algae. The medicament is useful as a therapeutic agent or prophylactic agent for a disease associated with bone such as osteoporosis or fracture. Moreover, an agent for enhancement of BMP production or an agent for promotion of osteogenesis can be used as a component of implant or dentifrice used for fracture healing or dental treatment. The agent is also useful for studies of functions of bone and screening of a drug for a disease associated with bones. In addition, the food or beverage will be able to ameliorate the symptoms of a disease requiring enhancement of BMP production or promotion of osteogenesis, or the like, by taking the food or beverage as daily foodstuff.

## Claims

1. A therapeutic agent or prophylactic agent for a disease requiring enhancement of bone morphogenetic protein production or promotion of osteogenesis, **characterized in that** the therapeutic agent or prophylactic agent comprises as an effective ingredient at least one compound selected from the group consisting of (a) an acidic saccharide, (b) a polyacrylic acid, (c) chlorogenic acid and (d) an oxidation processed product of chlorogenic acid.

2. The therapeutic agent or prophylactic agent according to claim 1, wherein the acidic saccharide is at least one acidic saccharide selected from the group consisting of an acidic polysaccharide, an acidic oligosaccharide, an acidic monosaccharide, and a degradation product thereof.

3. The therapeutic agent or prophylactic agent according to claim 1, wherein the acidic saccharide is at least one acidic saccharide selected from the group consisting of fucoidan, heparin, a dextran sulfate, chondroitin sulfate B, pectic acid, an acidic polysaccharide derived from *Spirulina,* a fucoidan degradation product, carrageenan λ, carrageenan κ, carrageenan 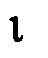 , a low-molecular weight heparin, a heparan sulfate and an acidic polysaccharide derived from *Chlorella.*

4. An agent for enhancement of bone morphogenetic protein production or an agent for promotion of osteogenesis, **characterized in that** the agent comprises as an effective ingredient at least one compound selected from the group consisting of (a) an acidic saccharide, (b) a polyacrylic acid, (c) chlorogenic acid and (d) an oxidation processed product of chlorogenic acid.

5. The agent for enhancement of bone morphogenetic protein production or the agent for promotion of osteogenesis according to claim 4, wherein the acidic saccharide is at least one acidic saccharide selected from the group consisting of an acidic polysaccharide, an acidic oligosaccharide, an acidic monosaccharide, and a degradation product thereof.

6. The agent for enhancement of bone morphogenetic protein production or the agent for promotion of osteogenesis according to claim 4, wherein the acidic saccharide is at least one acidic saccharide selected from the group consisting of fucoidan, heparin, a dextran sulfate, chondroitin sulfate B, pectic acid, an acidic polysaccharide derived from *Spirulina,* a fucoidan degradation product, carrageenan λ, carrageenan κ, carrageenan 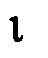 , a low-molecular weight heparin, a heparan sulfate and an acidic polysaccharide derived from *Chlorella.*

7. A food, beverage or feed for enhancement of bone morphogenetic protein production or promotion of osteogenesis, **characterized in that** the food, beverage or feed comprises as an effective ingredient at least one compound selected from the group consisting of (a) an acidic saccharide, (b) a polyacrylic acid, (c) chlorogenic acid and (d) an oxidation processed product of chlorogenic acid.

8. The food, beverage or feed for enhancement of bone morphogenetic protein production or promotion of osteogenesis according to claim 7, wherein the acidic saccharide is at least one acidic saccharide selected from the group consisting of an acidic polysaccharide, an acidic oligosaccharide, an acidic monosaccharide, and a degradation product thereof.

9. The food, beverage or feed for promotion of osteogenesis or enhancement of bone morphogenetic protein production according to claim 7, wherein the acidic saccharide is at least one acidic saccharide selected from the group consisting of fucoidan, heparin, a dextran sulfate, chondroitin sulfate B, pectic acid, an acidic polysaccharide derived from *Spirulina,* a fucoidan degradation product, carrageenan λ, carrageenan κ, carrageenan 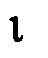 , a low-molecular weight heparin, a heparan sulfate and an acidic polysaccharide derived from *Chlorella.*

10. A therapeutic agent or prophylactic agent for a disease requiring enhancement of bone morphogenetic protein production or promotion of osteogenesis, **characterized in that** the therapeutic agent or prophylactic agent comprises as an effective ingredient an extract derived from algae.

11. An agent for enhancement of bone morphogenetic protein production or an agent for promotion of osteogenesis, **characterized in that** the agent comprises as an effective ingredient an extract derived from algae.

12. A food, beverage or feed for enhancement of bone morphogenetic protein production or promotion of osteogenesis, **characterized in that** the food, beverage or feed comprises as an effective ingredient an extract derived from algae.
